# EUROPÄISCHE PATENTSCHRIFT

(11) **EP 4 076 308 B1**
(45) Veröffentlichungstag und Bekanntmachung des Hinweises auf die Patenterteilung: **07.06.2023**
(21) Anmeldenummer: 21811356.1
(22) Anmeldetag: 18.11.2021
(51) Int. Cl.: A61F 9/007, A61M 5/20, A61M 5/24, A61M 5/30

(54) **STIFTARTIGE SPRITZENVORRICHTUNG UND SPRITZENSYSTEM**
PEN-LIKE SYRINGE DEVICE AND SYRINGE SYSTEM
DISPOSITIF À SERINGUE DE TYPE STYLO ET SYSTÈME À SERINGUE

(30) Priorität: 30.11.2020 DE 202020106870 U
(43) Veröffentlichungstag der Anmeldung: 26.10.2022
(73) Patentinhaber: PHARMPUR GmbH, 86343 Königsbrunn (DE)
(72) Erfinder: MENZ, Dirk-Henning, 86420 Diedorf (DE); GANSER, Klaus, 82205 Gilching (DE)
(74) Vertreter: Charrier Rapp & Liebau
(86) Internationale Anmeldenummer: PCT/EP2021/082143
(87) Internationale Veröffentlichungsnummer: WO 2022/112088

(56) Entgegenhaltungen:
- DE-T2- 69 525 567
- US-A1- 2009 299 328

## Beschreibung

Die Erfindung betrifft eine stiftartige Spritzenvorrichtung sowie ein Spritzensystem mit einer stiftartigen Spritzenvorrichtung und einer externen Versorgungseinheit.

In der Medizin, insbesondere im Bereich der Ophthalmologie, kommen Spritzenvorrichtungen bzw. Spritzensysteme mit einer Spritzenvorrichtung und einer mit dieser verbundenen externen Versorgungseinheit zur Injektion oder Entnahme von Substanzen in zahlreichen und unterschiedlichen Anwendungsbereichen zum Einsatz. Neben allgemein zu erfüllenden Anforderungen, die beispielsweise die Zuverlässigkeit und Sicherheit einer Injektion oder Entnahme gewährleisten sollen, sind dabei auch die Bedienerfreundlichkeit, speziell ein sicheres und vorteilhaftes Halten, Führen und Betätigen der Spritzenvorrichtung von Bedeutung.

Aus DE 695 25 567 T2 ist beispielsweise eine Zufuhrvorrichtung zum Zuführen eines injizierbaren Materials aus einer in die Zufuhrvorrichtung einbringbaren Spritze zu einer Injektionsstelle bekannt. Die Spritze umfasst dabei einen Körperbereich und einen in diesem bewegbar angeordneten Kolben. Die Zufuhrvorrichtung ist mit einer pneumatischen Zufuhr, insbesondere einer CO₂-Flasche, verbunden, wobei dem Kolben der Spritze über eine Steuerventilbaugruppe der Zufuhrvorrichtung ein unter Druck stehendes Fluid zugeführt werden kann. Die Steuerventilbaugruppe umfasst hierfür ein Stromsteuerventil und ein Dreiwegeventil, die durch einen Auslöser betätigt werden. Das Dreiwegeventil ist dabei insbesondere dazu ausgelegt, den Durchlass von Fluid zu erlauben, wenn es offen ist, und Fluiddruck von der stromabwärtigen Seite des Dreiwegeventils zu entlüften, wenn es geschlossen ist. Hierfür weist das Dreiwegeventil einen Stößel mit einem Entspannungsdurchlass auf, der selektiv zur Verbindung der Spritzenkolben-Seite des Dreiwegeventils mit der Atmosphäre verwendet werden kann.

Eine weitere Injektionsvorrichtung zur Injektion von Substanzen in das Gewebe eines Patienten ist aus US 2009/0299328 A1 bekannt.

Es besteht die Aufgabe, eine ergonomische Spritzenvorrichtung für medizinische und insbesondere für ophthalmologische Zwecke, insbesondere zum Einbringen von Fluiden in das menschliche oder tierische Auge, sowie ein Spritzensystem mit einer Spritzenvorrichtung und einer mit dieser verbundenen externen Versorgungseinheit bereitzustellen, die ein einfaches und sicheres Halten, Führen und Bedienen der Spritzenvorrichtung, insbesondere auch unabhängig von der Größe der Hand eines Bedieners, eine sichere und gezielte Injektion einer Substanz oder die Entnahme einer Substanz ermöglicht, insbesondere eine Injektion in ein menschliches oder tierisches Auge bzw. eine Entnahme einer Substanz aus einem Auge. Weiter soll eine Kontamination der zu injizierenden bzw. zu entnehmenden Substanz und/oder des Auges verhindert werden.

Unter einer Spritze ist hierbei eine Vorrichtung zu verstehen, die einen Spritzenkörper (bzw. Primärcontainer), insbesondere einen zylindrischen Spritzenkörper, mit einer an seinem vorderen (proximalen) Ende angeordneten Ejektionsvorrichtung, beispielsweise eine Düse und/oder eine Kanüle, aufweist und einen in dem Spritzenkörper beweglich geführten Kolben (ohne Kolbenstange) bzw. einen Spritzenstopfen enthält. Der Begriff Spritze umfasst dabei auch sogenannte Carpulen und Zylinderampullen mit einer durchstechbaren Membran an ihrem vorderen (proximalen) Ende.

Gelöst wird die Aufgabe mit einer stiftartigen Spritzenvorrichtung mit den Merkmalen des Anspruchs 1 sowie einem Spritzensystem mit den Merkmalen des Anspruchs 14. Zweckmäßige Ausgestaltungen und vorteilhafte Weiterbildungen der Erfindung sind den Unteransprüchen entnehmbar.

Unter einer stiftartigen Spritzenvorrichtung ist eine im Wesentlichen länglich ausgestaltete Spritzenvorrichtung zu verstehen, die von einem Bediener wie ein Stift in einer Hand gehalten werden kann. Die stiftartige Spritzenvorrichtung wird dabei üblicherweise durch zwei Finger (wobei der Begriff Finger im Folgenden auch den Daumen umfasst), insbesondere zwischen Daumen und Zeigefinger, oder durch drei Finger, insbesondere zwischen Daumen, Zeigefinger und Mittelfinger, gehalten, geführt und bedient.

Die erfindungsgemäße stiftartige Spritzenvorrichtung umfasst ein Antriebsgehäuse, eine an das Antriebsgehäuse anschließende Spritzenaufnahme zur Aufnahme einer Spritze sowie einen manuell betätigbaren Taster zur Aktivierung einer elektrischen Fördereinrichtung, insbesondere zur Aktivierung einer in dem Antriebsgehäuse angeordneten elektrischen Fördereinrichtung oder zur Aktivierung einer elektrischen Fördereinrichtung, die in einer mit der stiftartigen Spritzenvorrichtung verbindbaren externen Versorgungseinheit angeordnet ist. Die Spritzenaufnahme stellt dabei einen vorderen (proximalen) Teil und das Anschlussgehäuse einen hinteren (distalen) Teil der stiftartigen Spritzenvorrichtung dar. Eine in die Spritzenaufnahme einbringbare Spritze weist einen Spritzenkörper, insbesondere einen zylindrischen Spritzenkörper, und einen in dem Spritzenkörper beweglich geführten Kolben bzw. Spritzenstopfen auf. An seinem vorderen (proximalen) Ende kann die Spritze eine konus-oder zylinderförmige Düse oder eine andere Ejektionsvorrichtung, wie z.B. eine Kanüle aufweisen, wobei an der Ejektionsvorrichtung ein Anschluss, insbesondere ein Luer-Anschluss (Luer-Lock, Luer-Slip) zur Verbindung mit einem Schlauch oder einer Injektionsnadel vorgesehen sein kann.

Das Antriebsgehäuse umfasst eine Druckversorgungseinheit zur pneumatischen Beaufschlagung des Kolbens einer in die Spritzenaufnahme eingebrachten Spritze mit Druck oder Unterdruck, insbesondere zur direkten bzw. unmittelbaren Beaufschlagung des Kolbens mit Druck oder Unterdruck, und einen Koppelanschluss zur pneumatischen und/oder elektrischen Verbindung der Druckversorgungseinheit mit einer externen Versorgungseinheit, welche entfernt vom Antriebsgehäuse angeordnet ist. Die elektrische Versorgungseinheit kann dabei eine Stromversorgungseinheit und/oder eine elektrische Fördereinrichtung, die über die Stromversorgungseinheit mit elektrischer Energie versorgt wird, aufweisen. Die Spritzenvorrichtung selbst weist keine integrierte Stromversorgungseinheit, wie eine Batterie oder einen Akkumulator, und/oder keine elektrische Fördereinrichtung auf.

Zur pneumatischen Beaufschlagung des Kolbens einer in die Spritzenaufnahme eingebrachten Spritze mit Druck oder Unterdruck umfasst die Druckversorgungseinheit einen Antriebskanal, der mit dem Kolben einer in die Spritzenaufnahme eingebrachten Spritze verbindbar ist. Insbesondere kann das Antriebsgehäuse dabei einen Verbindungsstutzen aufweisen, der zumindest ein kleines Stück in den Spritzenkörper einer eingebrachten Spritze hineinragen kann und durch welchen der Antriebskanal bzw. ein Abschnitt des Antriebskanals verläuft. Weiter umfasst die stiftartige Spritzenvorrichtung einen Entlüftungskanal mit einem ersten Ende, das mit dem Antriebskanal verbunden ist, und einem zweiten Ende, das über eine Öffnung mit der Umgebung verbunden ist. Die Öffnung ist bei Betätigung des Tasters von einem den Taster betätigenden Finger verschließbar.

Durch die Auslagerung der Stromversorgungseinheit und/oder der elektrischen Fördereinrichtung kann das Gewicht der stiftartigen Spritzenvorrichtung gegenüber einer Spritzenvorrichtung mit integrierter Stromversorgungseinheit und/oder elektrischer Fördereinrichtung deutlich reduziert werden, wodurch die Handhabung der stiftartigen Spritzenvorrichtung wesentlich angenehmer ist und eine Belastung der Hand eines Bedieners reduziert wird. Insbesondere kann dabei vermieden werden, dass durch Anordnung der elektrischen Fördereinrichtung und/oder der Stromversorgung im oder am Antriebsgehäuse die Gewichtsverteilung der stiftartigen Spritzenvorrichtung "hecklastig" ist, d.h. dass der hintere Bereich der stiftartigen Spritzenvorrichtung im Vergleich zu dem vorderen Bereich zu schwer ist, wodurch die Handhabung der stiftartigen Spritzenvorrichtung unvorteilhaft und unangenehm wäre. Dies kann insbesondere beim Einsatz von großen und leistungsstarken elektrischen Fördereinrichtungen sowie schweren Stromversorgungseinheiten und Batterien oder Akkumulatoren der Fall sein. Besonders bevorzugt weist die stiftartige Spritzenvorrichtung weder eine Stromversorgungseinheit, insbesondere keine Batterien oder Akkumulatoren, noch eine elektrische Fördereinrichtung auf. Die elektrische Fördereinrichtung und deren Stromversorgung ist in diesem Fall in der externen Versorgungseinheit integriert und diese ist bspw. über einen Schlauch mit dem Koppelanschluss des Anschlussgehäuses der stiftartigen Spritzenvorrichtung pneumatisch verbunden. Durch die Auslagerung der Stromversorgungseinheit und der elektrischen Fördereinrichtung auf die externe Versorgungseinheit ist eine einfach zu sterilisierende stiftartige Spritzenvorrichtung gegeben. Dabei muss insbesondere keine Rücksichtnahme auf die empfindliche elektrische Fördereinrichtung und die Stromversorgungseinheit genommen werden, wenn die stiftartigen Spritzenvorrichtung gereinigt oder sterilisiert werden muss, weshalb Beschädigungen der elektrischen Fördereinrichtung und der Stromversorgungseinheit vermieden werden können.

Die stiftartige Spritzenvorrichtung kann dabei auch als kostengünstiges Wegwerfteil, das nach einmaliger oder nach wenigen Benutzungen entsorgt wird, konzipiert sein. Eine aufwendige Sterilisation der externen Versorgungseinheit ist nicht zwingend erforderlich, da diese entfernt von der stiftartigen Spritzenvorrichtung und somit entfernt von einem Patienten, beispielsweise an einem Arm eines Bedieners angeordnet und von sterilen Materialien, beispielsweise der OP-Kleidung des Bedieners oder von einer Arm- oder Handgelenkstulpe, abgedeckt werden kann. Eine einfache Desinfizierung der externen Versorgungseinheit nach Benutzung ist in diesem Fall ausreichend und auf eine Sterilisation kann in diesem Fall gänzlich verzichtet werden.

Die stiftartige Spritzenvorrichtung kann insbesondere für medizinische Zwecke verwendet werden, speziell für die Ophthalmologie. Hierfür ist das Spritzensystem zweckmäßig aus für medizinische Zwecke geeigneten, sterilen oder strerilisierbaren Materialien, wie Aluminium, Edelstahl, sterilisationsstabilen Kunststoffen oder Kombinationen aus diesen, hergestellt. Dies ermöglicht eine einfache Sterilisation, beispielsweise durch Dampf oder Röntgen- oder UV-Bestrahlung oder Gammastrahlen oder Ethylenoxid.

Durch die erfindungsgemäße stiftartige Spritzenvorrichtung ist eine ergonomische Spritzenvorrichtung gegeben, die neben einem einfachen und sicheren Halten und Führen auch ein einfaches und zielsicheres Bedienen der stiftartigen Spritzenvorrichtung, insbesondere auch unabhängig von der Größe der Hand eines Bedieners, ermöglicht. Die stiftartige Spritzenvorrichtung gewährleistet eine sichere und gezielte Injektion einer Substanz oder die Entnahme einer Substanz, insbesondere in bzw. aus einem menschlichen oder tierischen Auge. Dabei kann die stiftartige Spritzenvorrichtung wie ein Stift zwischen Daumen und Zeigefinger bzw. zwischen Daumen, Zeigefinger und Mittelfinger gehalten werden. Das Halten, Führen und Bedienen der stiftartigen Spritzenvorrichtung kann dabei insbesondere nahe des von dem Antriebsgehäuse abweisenden vorderen Endes einer in die Spritzenaufnahme eingebrachten Spritze, bspw. nahe einer Kanüle der Spritze, und somit nahe am Injektions- oder Ejektionsort, beispielsweise in einem zu behandelnden Auge erfolgen. Dadurch ist eine sichere und gute Führung der stiftartigen Spritzenvorrichtung bzw. eine einfache Handhabung der stiftartigen Spritzenvorrichtung gegeben. Eine gegebenenfalls erforderliche Änderung einer Position der stiftartigen Spritzenvorrichtung während einer Injektion bzw. während einer Entnahme, speziell eine Änderung der Position der Spitze einer Kanüle, beispielsweise durch Zurückziehen oder Einbringen der Kanülenspitze in menschliches oder tierisches Gewebe, ist dabei auf einfache und vorteilhafte Weise möglich. Eine Positionsänderung der stiftartigen Spritzenvorrichtung bzw. einer Ejektionsvorrichtung wie einer Kanülenspitze ist beispielsweise im Bereich der Ophthalmologie erforderlich, wenn Färbemittel oder eine Perfluorcarbon- oder Silikonölblase im Auge platziert werden soll.

Bei Betätigen des Tasters erfolgt eine Aktivierung der in der externen Versorgungseinheit oder gegebenenfalls einer in dem stiftartigen Spritzensystem, speziell dem Antriebsgehäuse, angeordneten elektrischen Fördereinrichtung, die einen Druck oder Unterdruck zur pneumatischen Beaufschlagung des Kolbens einer in die Spritzenaufnahme eingebrachten Spritze mit Druck oder Unterdruck bereitstellt. Eine Betätigung des Tasters kann insbesondere über den Zeigefinger erfolgen. Für eine Injektion bzw. Entnahme einer Substanz ist lediglich die zur Betätigung des Tasters erforderliche Kraft aufzubringen. Im Vergleich zu herkömmlichen Spritzen, bei denen eine manuelle Betätigung einer mit dem Kolben der Spritze verbundenen Kolbenstange erforderlich ist, weist die stiftartige Spritzenvorrichtung eine einfache und angenehme Bedienbarkeit auf. Insbesondere ist zur Betätigung der stiftartigen Spritzenvorrichtungs keine große Kraft aufzubringen bzw. kein starkes Drücken erforderlich. Dadurch ist ein sicheres Halten und Führen der stiftartigen Spritzenvorrichtung, insbesondere auch während einer Injektion oder Entnahme, gegeben. Es ist auch denkbar, dass die zur Betätigung des Tasters aufzubringende Kraft über eine an der stiftartigen Spritzenvorrichtung angeordnete Vorrichtung einstellbar ist, um beispielsweise eine gewünschte Aktivierungskraft einzustellen.

Bei der erfindungsgemäßen stiftartigen Spritzenvorrichtung ist, abgesehen von der Beaufschlagung des Kolbens mit Druck oder Unterdruck, keine weitere Medienverbindung mit dem Kolben, beispielsweise durch eine Kolbenstange oder durch ein hydraulisches Fördermedium etc., gegeben. Die Gefahr einer möglichen Kontamination einer zu injizierenden oder zu entnehmenden Substanz, die abgesehen von dem Inneren des Spritzenkörpers lediglich mit dem Kolben der Spritze in Verbindung steht, besteht dabei nicht.

Weiter ermöglicht die Ausgestaltung der stiftartigen Spritzenvorrichtung, insbesondere die Beaufschlagung des Kolbens einer in die Spritzenaufnahme eingebrachten Spritze mit Druck oder Unterdruck durch die über den Taster aktivierbare elektrische Fördereinrichtung, ein exaktes Dosieren der Injektion bzw. der Entnahme einer Substanz. Durch die elektrische Fördereinrichtung kann dabei insbesondere langsam ein Druck oder Unterdruck aufgebaut werden, wodurch eine schlagartige und unkontrollierte Injektion oder Entnahme verhindert wird. Weiter ist eine Injektion in beliebigen Dosen, bspw. tropfenweise oder im Dauerstrom, und insbesondere eine Feindosierung möglich. Die Dosierung kann also vom Operateur unmittelbar und situationsgerecht auf den tatsächlichen Bedarf angepasst werden, ohne dass entsprechende Parameter vorab eingestellt oder programmiert werden müssen. Insbesondere ist dabei auch eine kontinuierliche Injektion ohne ein bei bekannten Spritzenvorrichtungen auftretendes Hakeln des Kolbens, bspw. aufgrund einer inhomogenen Silikonisierung der Spritzen und/oder aufgrund eines Festklebens bzw. Festsetzens des Kolbens am Spritzenkörper, gewährleistet. Entsprechendes gilt auch für die Entnahme einer Substanz.

Die Beaufschlagung des Kolbens einer in die Spritzenaufnahme der erfindungsgemäßen stiftartigen Spritzenvorrichtung eingebrachten Spritze mit Druck oder Unterdruck erfolgt nur, wenn die an dem zweiten Ende des Entlüftungskanals angeordnete Öffnung verschlossen ist. Andernfalls steht der Antriebskanal über den Entlüftungskanal mit der Umgebung in Verbindung. Eine Beaufschlagung des Kolbens mit Druck oder Unterdruck und somit eine Bewegung des Kolbens relativ zu dem Spritzenkörper wird dadurch unterbunden bzw. unmittelbar beendet. Die Ausgestaltung der stiftartigen Spritzenvorrichtung gewährleistet somit ein unmittelbares bzw. sofortiges Beenden einer Injektion oder Entnahme sobald die Betätigung des Tasters beendet bzw. die Öffnung des Entlüftungskanals nicht oder nicht mehr verschlossen ist. Über den Entlüftungskanal erfolgt eine sofortige Entspannung eines zuvor auf den Kolben wirkenden Drucks oder Unterdrucks. Ein mögliches "Nachlaufen" oder Nachtropfen einer Injektion oder einer Entnahme einer Substanz ist somit nicht möglich. Weiter ist bei einer nicht vorgesehenen bzw. nicht ordnungsgemäßen Betätigung des Tasters, beispielsweise bei einer ungewollten Berührung des Tasters oder beispielsweise bei nicht korrekter Platzierung des zur Betätigung des Tasters vorgesehenen Fingers bzw. einer nicht ordnungsgemäßen Handhabung des Spritzensystems, die Öffnung des Entlüftungskanals nicht verschlossen. Eine ungewollte oder unkontrollierte Beaufschlagung des Kolbens mit Druck oder Unterdruck bzw. eine ungewollte oder unkontrollierte Injektion oder Entnahme wird somit unterbunden.

In einer bevorzugten Ausgestaltung der stiftartigen Spritzenvorrichtung ist die Öffnung direkt an dem Taster, beispielsweise auf der Oberseite des Tasters, oder unmittelbar neben dem Taster angeordnet. Die Öffnung ist dadurch unmittelbar durch einen den Taster betätigenden Finger, speziell durch den betätigenden Finger selbst, beispielsweise den Zeigefinger eines Bedieners, verschließbar. Eine unmittelbare Anordnung der Öffnung neben dem Taster entspricht dabei einer Anordnung der Öffnung so nahe bei dem Taster, dass diese durch den den Taster bedienenden Finger, insbesondere das dritte bzw. vorderste Fingerglied (Endglied), bei einer Betätigung des Tasters verschließbar ist. Die Öffnung des Entlüftungskanals ist dabei insbesondere so platziert, dass der Bediener diese bei ordnungsgemäßer Betätigung des Tasters und ordnungsgemäßer Handhabung des Spritzensystems zwingend verschließt. Durch die beschriebene Ausgestaltung ist das beschriebene unmittelbare und sofortige Beenden einer Injektion oder Entnahme gewährleistet und eine ungewollte oder unkontrollierte Beaufschlagung des Kolbens mit Druck oder Unterdruck wird unterbunden.

In einer bevorzugten Ausgestaltung enthält die externe Versorgungseinheit eine elektrische Fördereinrichtung zur Versorgung der Druckversorgungseinheit mit Druck oder Unterdruck. Zweckmäßigerweise enthält die externe Versorgungseinheit dabei auch eine Stromversorgungseinheit für die elektrische Fördereinrichtung. Die stiftartige Spritzenvorrichtung selbst weist dabei keine elektrische Fördereinrichtung und auch keine Stromversorgungseinheit auf. Die Versorgung der Druckversorgungseinheit mit Druck oder Unterdruck erfolgt hierbei durch die in der externen Versorgungseinheit angeordnete elektrische Fördereinrichtung, die bspw. über einen Schlauch pneumatisch mit dem Koppelanschluss des Antriebsgehäuses verbunden werden kann, welcher mit dem Antriebskanal pneumatisch in Verbindung steht. Durch die Anordnung der elektrischen Fördereinrichtung und auch der Stromversorgungseinheit in der externen Versorgungseinheit ist eine auf das Gewicht bezogen besonders leichte Ausgestaltung der stiftartigen Spritzenvorrichtung gegeben.

Besonders bevorzugt ist die elektrische Fördereinrichtung über den Taster der stiftartigen Spritzenvorrichtung betätigbar, wobei bei Betätigung des Tasters ein Beaufschlagen des Antriebskanals mit Druck oder Unterdruck erfolgt. Hierfür kann eine elektrische Verbindung zwischen dem Taster und der elektrischen Fördereinrichtung bzw. der externen Versorgungseinheit vorgesehen sein. Besonders bevorzugt umfasst die elektrische Fördereinrichtung eine Membranpumpe. Die Beaufschlagung des Kolbens einer in die Spritzenaufnahme eingebrachten Spritze erfolgt dabei zweckmäßig mit Druckluft bzw. einem Unterdruck gegenüber der Atmosphäre. Je nach Bedarf an bereitzustellendem Druck oder Unterdruck kann die elektrische Fördereinrichtung dabei auch mehrere, insbesondere zwei Membranpumpen, umfassen. Durch den Einsatz einer Membranpumpe und den Einsatz von Luft als antreibendes Medium ist die Betätigung des Kolbens auf besonders einfache und vorteilhafte Weise möglich. Eine mechanische Vorrichtung, die zur Bewegung des Kolbens mit diesem in Verbindung steht, ist hierbei nicht erforderlich. Durch den Einsatz der Membranpumpe und das Beaufschlagen des Kolbens mit Druckluft bzw. mit Unterdruck entfallen somit aufwendige und fehleranfällige Getriebe zwischen der Fördereinrichtung und dem Kolben. Weiter sind Membranpumpen sehr robust und unempfindlich gegenüber Störungen.

In einer alternativen Ausgestaltung kann die Druckversorgungseinheit der Spritzenvorrichtung eine mit dem Antriebskanal verbundene elektrische Fördereinrichtung, insbesondere eine Membranpumpe, umfassen, wobei durch Betätigen des Tasters eine Aktivierung der elektrischen Fördereinrichtung erfolgt. Die elektrische Fördereinrichtung ist dabei im Antriebsgehäuse der stiftartigen Spritzenvorrichtung integriert und somit Teil der stiftartigen Spritzenvorrichtung. Die Stromversorgungseinheit der elektrischen Fördereinrichtung ist hingegen in der externen Versorgungseinheit angeordnet, wobei die elektrische Fördereinrichtung über den Koppelanschluss mit der elektrischen Fördereinrichtung elektrisch verbunden ist. Eine derartige Ausgestaltung bietet sich insbesondere für stiftartige Spritzenvorrichtungen und deren Anwendungszwecke an, bei denen kleine und leichte elektrische Fördereinrichtungen eingesetzt werden können, beispielsweise wenn die stiftartige Spritzenvorrichtung zur Aufnahme von kleinen 2,5 ml Standardspritzen vorgesehen ist, und die Gewichtseinsparung durch die Auslagerung der Stromversorgungseinheit auf eine externe Versorgungseinheit ausreichend ist. Durch die beschriebene Anordnung der in der stiftartigen Spritzenvorrichtung integrierten elektrischen Fördereinrichtung sind konstruktionstechnische vorteilhafte Ausgestaltungen, wie eine kurze elektrische Verbindung zwischen Taster und elektrischer Fördereinrichtung sowie eine kurze Ausgestaltung des Antriebskanals, möglich.

Bevorzugt ist der Taster nahe dem vorderen bzw. dem von dem Antriebsgehäuse wegweisenden Ende der stiftartigen Spritzenvorrichtung angeordnet. Die dadurch gegebene Anordnung der zur Bedienung der stiftartigen Spritzenvorrichtung verwendeten Finger nahe dem vorderen Ende der Spritze, beispielsweise nahe einer an der Spritze angebrachten Kanüle, ermöglicht eine sichere Führung des Spritzensystems, insbesondere bei gleichzeitiger Betätigung des Tasters.

In einer vorteilhaften Ausgestaltung der stiftartigen Spritzenvorrichtung kann die Spritzenaufnahme einen schwenkbar mit dem Antriebsgehäuse verbundenen Bedienhebel umfassen bzw. unter Verwendung von diesem gebildet sein, wobei der Bedienhebel bevorzugt über ein an einem von der Spritzenaufnahme wegweisenden hinteren Bereich des Antriebsgehäuses angeordneten Drehgelenk mit dem Antriebsgehäuse verbunden ist. Durch Schwenken des mit dem Antriebsgehäuse verbundenen Bedienhebels ist dabei ein zügiges und einfaches Einbringen einer Spritze in die sowie ein Ausbringen der Spritze aus der Spritzenaufnahme auf vorteilhafte Weise möglich. Besonders bevorzugt verläuft der Entlüftungskanal dabei zumindest teilweise in dem Bedienhebel. Weiter können der Taster und die Öffnung an bzw. auf dem Bedienhebel angeordnet sein. Durch die beschriebene Ausgestaltung ist eine konstruktionstechnisch vorteilhafte Ausgestaltung der stiftartigen Spritzenvorrichtung gegeben. Beispielsweise sind dabei zusätzliche Bauteile, die den Entlüftungskanal bilden oder den Taster und die Öffnung tragen, nicht erforderlich.

Bevorzugt umfasst die Spritzenaufnahme eine Kopfeinheit, insbesondere eine an einem vorderen Bereich eines Bedienhebels angeordnete Kopfeinheit. Die Kopfeinheit fasst dabei zumindest teilweise die Spritzenschulter einer in die Spritzenaufnahme eingebrachten Spritze. Die Spritzenschulter ist dabei der vordere (proximale) Bereich einer Spritze, der den Übergangsbereich von dem zylindrischen Spritzenkörper zu der Düse bzw. der Anschlussvorrichtung bildet. Im Betriebszustand der stiftartigen Spritzenvorrichtung liegt die Spritzenschulter bzw. zumindest ein Teil der Spritzenschulter dabei unmittelbar an der Kopfeinheit an. Besonders bevorzugt ist die in die Spritzenaufnahme eingebrachte Spritze im Betriebszustand der stiftartigen Spritzenvorrichtung durch die Kopfeinheit und die in Richtung der Spritzenaufnahme weisenden Seite des Antriebsgehäuses eingeklemmt und fixiert. Bevorzugt ist hierbei auf der in Richtung der Spritzenaufnahme weisenden Seite des Antriebsgehäuses ein Dichtelement, speziell ein Dichtring, angeordnet, der an der Spritze bzw. einem Spritzenflansch einer Spritze anliegt, wodurch eine gas- oder luftdichte Verbindung zwischen Spritze und Antriebsgehäuse gegeben ist. Der Spritzenflansch stellt hierbei das hintere (distale) Ende der Spritze, speziell ein den zylindrischen Spritzenkörper umlaufender, vorstehender Rand dar. Bei der beschriebenen Ausgestaltung ist insbesondere kein Klemmen eines Spritzenflansches, also eine Fixierung der Spritze durch beidseitiges Klemmen des Spritzenflansches, erforderlich. Ein beidseitiges Klemmen des Spritzenflansches führt oftmals zur Beschädigung oder zum Abbruch des Spritzenflansches, aufgrund fertigungstechnischer Toleranzen bei denen oftmals der Spritzenflansch ungewollt etwas in Richtung des vorderen Endes der Spritze geneigt ist.

In einer bevorzugten Ausgestaltung kann die stiftartige Spritzenvorrichtung, insbesondere die Spritzenaufnahme, eine Temperiereinheit, speziell eine Heizeinheit, umfassen. Die Temperiereinheit dient dabei zur Temperierung einer in einer eingebrachten Spritze enthaltenen, zu injizierenden Substanz. Die Temperiereinheit kann insbesondere in dem Bedienhebel und/oder der Kopfeinheit angeordnet sein. Vorzugsweise wird die zu injizierende Substanz dabei auf Körpertemperatur erwärmt. Eine Temperierung führt dabei zu einer Verringerung der Viskosität der zu injizierenden Substanz, wodurch eine Injektion begünstigt wird. Die Versorgung der Temperiereinheit mit elektrischer Energie erfolgt dabei bevorzugt über eine außerhalb der stiftartigen Spritzenvorrichtung angeordnete Stromversorgungseinheit.

Weiter umfasst die Erfindung ein Spritzensystem mit einer oben beschriebenen stiftartigen Spritzenvorrichtung und einer entfernt vom Antriebsgehäuse angeordneten externen Versorgungseinheit, die eine Stromversorgungseinheit, insbesondere eine Batterie oder einen Akkumulator, und/oder eine elektrische Fördereinrichtung, insbesondere zumindest eine Membranpumpe, umfasst und über den Koppelanschluss mit der stiftartigen Spritzenvorrichtung verbindbar oder verbunden ist. Die Stromversorgungseinheit kann dabei auch die oben erwähnte Temperiereinheit mit elektrischem Strom versorgen.

In einer bevorzugten Ausgestaltung, in der die externe Versorgungseinheit sowohl eine elektrische Fördereinrichtung als auch eine Stromversorgungseinheit zur Versorgung der elektrischen Fördereinrichtung und/oder der Temperiereinheit mit elektrischer Energie umfasst, weist die stiftartige Spritzenvorrichtung weder eine elektrische Fördereinrichtung noch eine Stromversorgungseinheit auf. Die elektrische Fördereinrichtung ist in diesem Fall über einen Schlauch mit dem Koppelanschluss der stiftartigen Spritzenvorrichtung, der mit dem Antriebskanal in Verbindung steht, verbunden. Die Betätigung der in der externen Versorgungseinheit angeordneten elektrischen Fördereinrichtung erfolgt bevorzugt über den Taster der stiftartigen Spritzenvorrichtung, wobei der Taster und die elektrische Fördereinrichtung über eine Betätigungsverbindung, die insbesondere durch den Bedienhebel, das Antriebsgehäuse entlang des Koppelanschlusses und gemeinsam mit dem Schlauch zur elektrischen Fördereinrichtung verlaufen kann. Die Betätigungsverbindung kann dabei insbesondere aus zwei miteinander koppelbaren Abschnitten bestehen, wobei der erste Abschnitt in der stiftartigen Spritzenvorrichtung und der zweite Abschnitt zwischen der stiftartigen Spritzenvorrichtung und der elektrischen Fördereinrichtung verläuft.

In einer alternativen Ausgestaltung, in der die externe Versorgungseinheit nur die Stromversorgungseinheit umfasst, ist die elektrische Fördereinrichtung in der stiftartigen Spritzenvorrichtung, genauer in dem Antriebsgehäuse der Spritzenvorrichtung angeordnet und Teil der Druckversorgungseinheit. Die Stromversorgungseinheit ist in diesem Fall über ein Kabel mit dem Koppelanschluss, der mit der elektrischen Fördereinrichtung in Verbindung steht, verbunden.

Besonders bevorzugt umfasst die externe Versorgungseinheit eine Befestigungseinrichtung, insbesondere eine Armmanschette oder einen Armreif, mit der die externe Versorgungseinheit an einem Arm eines Bedieners befestigbar ist. Dadurch kann die externe Versorgungseinheit auf besonders einfache Weise an einem Bediener, insbesondere dessen Arm, angebracht werden. Durch die Anordnung der externen Versorgungseinheit an dem Arm eines Bedieners verläuft die Verbindung zwischen der externen Versorgungseinheit und der stiftartigen Spritzenvorrichtung, beispielsweise ein Schlauch oder ein Kabel, derart, dass die Handhabung der stiftartigen Spritzen nicht störend beeinträchtigt wird. Außerdem kann die externe Versorgungseinheit dabei beispielsweise durch die Kleidung eines Bedieners, beispielsweise den Ärmel eines OP-Kittels oder eine Arm- oder Handgelenkstulpe, auf einfache Weise abgedeckt werden. Eine Sterilisation der externen Versorgungseinheit nach einer Benutzung ist dabei nicht oder zumindest nicht zwingend erforderlich. Insbesondere kann eine einfache Desinfizierung der externen Versorgungseinheit ausreichen.

In einer besonders bevorzugten Ausgestaltung weist die externe Versorgungseinheit einen Tuchspender, insbesondere einen Tuchspender mit einer bevorzugt textilen Abdeckung zur Abdeckung der externen Versorgungseinheit auf. Dadurch ist ein besonders einfaches Abdecken der Versorgungseinheit durch eine an der externen Versorgungseinheit selbst angeordnete textile Abdeckung gegeben.

Die externe Versorgungseinheit kann weiter eine Steuereinheit zur Steuerung der externen Versorgungseinheit, insbesondere zur Steuerung einer elektrischen Fördereinrichtung der externen Versorgungseinheit aufweisen. Zusätzlich oder alternativ kann die externe Versorgungseinheit eine Anzeigeeinheit zur Anzeige von Parametern des Spritzensystems, insbesondere des Zustands der Stromversorgungseinheit und/oder der Betriebsparameter der elektrischen Fördereinrichtung und/oder des auf den Kolben einer in die Spritzenaufnahme eingebrachten Spritze wirkenden Drucks oder Unterdrucks, aufweisen. Dadurch ist eine optimale Steuerung und Überwachung der externen Versorgungseinheit und/oder der stiftartigen Spritzenvorrichtung möglich.

Diese und weitere Merkmale sowie Vorteile und Wirkungen des erfindungsgemäßen stiftartigen Spritzensystems ergeben sich aus den nachfolgenden unter Bezugnahme auf die begleitenden Zeichnungen näher beschriebenen Ausführungsbeispiele. Die Zeichnungen zeigen:
- **Fig. 1**: ein Spritzensystem mit einer stiftartigen Spritzenvorrichtung und einer externen Versorgungseinheit gemäß einem ersten Ausführungsbeispiel;
- **Fig. 2**: eine Seitenansicht der stiftartigen Spritzenvorrichtung aus Fig. 1 in geöffneter Stellung;
- **Fig. 3**: eine Seitenansicht der stiftartigen Spritzenvorrichtung aus Fig. 1 in geschlossener Stellung mit eingebrachter Spritze;
- **Fig. 4**: eine Draufsicht auf die stiftartige Spritzenvorrichtung aus Fig. 1 in geschlossener Stellung mit eingebrachter Spritze;
- **Fig. 5**: eine Schnittansicht entlang einer Längsachse der stiftartigen Spritzenvorrichtung aus Fig. 1 in geschlossener Stellung mit eingebrachter Spritze;
- **Fig. 6**: die externe Versorgungseinheit des Spritzensystems aus Fig. 1 ohne Deckel;
- **Fig. 7**: das Spritzensystem aus Fig. 1 mit skizzierter Armmanschette;
- **Fig. 8**: ein Spritzensystem mit einer stiftartigen Spritzenvorrichtung und einer externen Versorgungseinheit gemäß einem zweiten Ausführungsbeispiel;
- **Fig. 9**: die externe Versorgungseinheit des Spritzensystems aus Fig. 8.

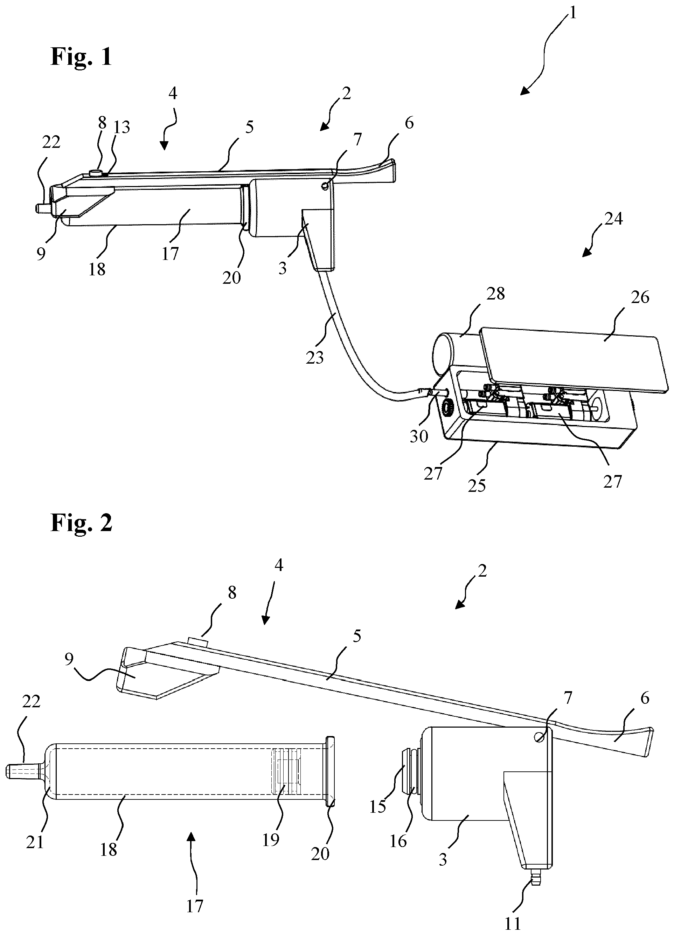

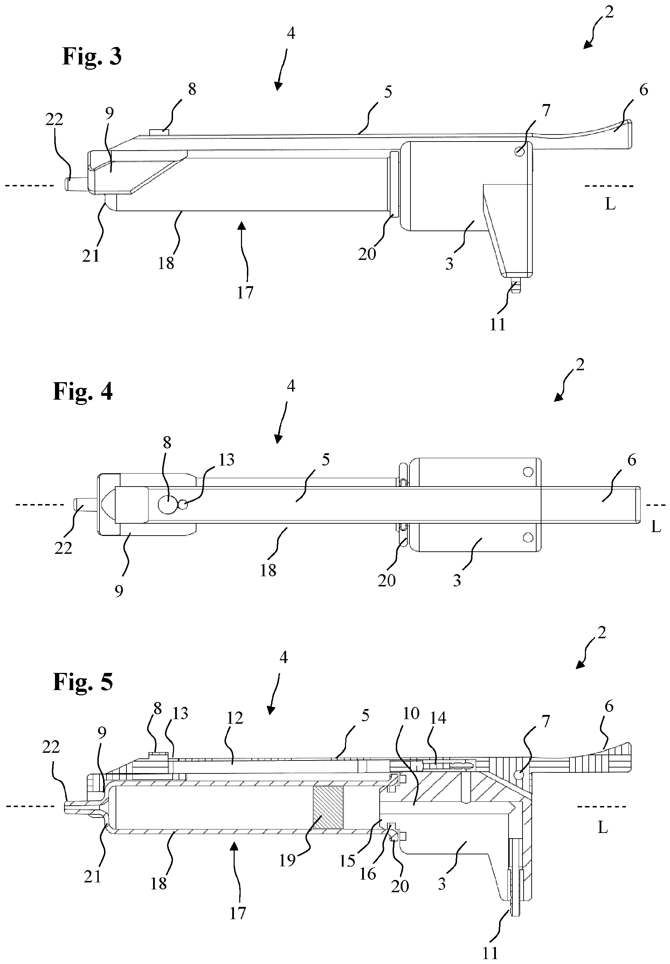

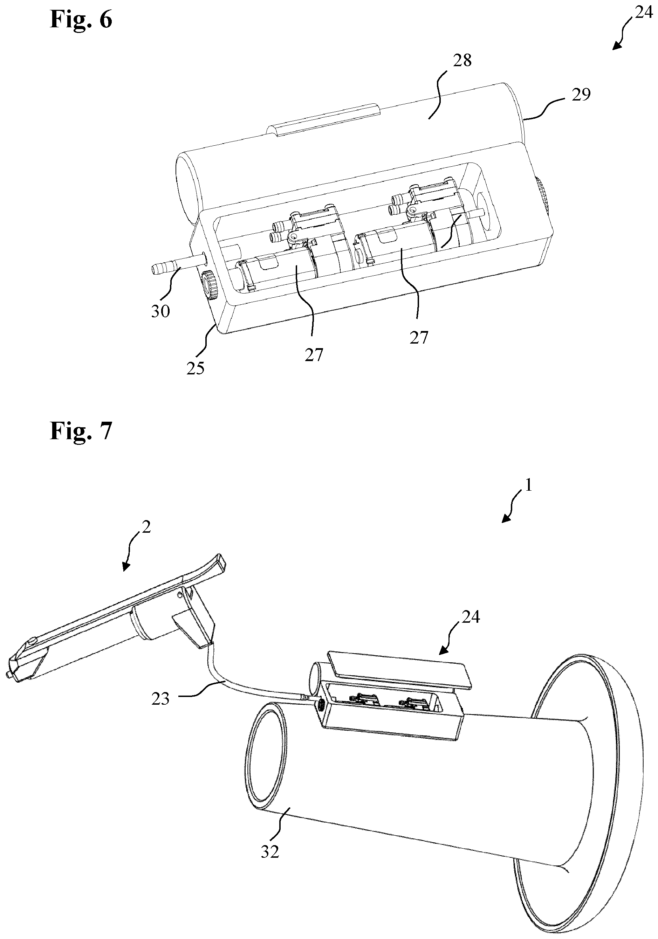

In Fig. 1 ist ein erstes Ausführungsbeispiel eines Spritzensystems 1 mit einer stiftartigen Spritzenvorrichtung 2 und einer externen Versorgungseinheit 24, die Druck oder Unterdruck für die stiftartige Spritzenvorrichtung 2 bereitstellen kann und über einen Schlauch 23 mit der stiftartigen Spritzenvorrichtung 2 verbunden ist, gezeigt. Die stiftartige Spritzenvorrichtung 2 ist in Fig. 2 in geöffneter Stellung und in den Fig. 3 bis 5 in geschlossener Stellung und mit eingebrachter Spritze 17, dem Betriebszustand der stiftartigen Spritzenvorrichtung 2, gezeigt. Die externe Versorgungseinheit 24 ist in Fig. 6 im Detail dargestellt.

Die stiftartige Spritzenvorrichtung 2 weist, wie insbesondere in den Fig. 2 bis 5 dargestellt ist, ein Antriebsgehäuse 3 und eine an das Antriebsgehäuse 3 anschließende Spritzenaufnahme 4 zur Aufnahme einer Spritze 17 auf, welche einen Spritzenkörper 18 mit einem an dem hinteren (distalen) Ende angeordneten Spritzenflansch 20 sowie einer an dem vorderen (proximalen) Ende angeordneten Düse 22 und einen in dem Spritzenkörper 18 beweglich geführten Kolben 19 bzw. Spritzenstopfen aufweist. Das Antriebsgehäuse 3 bildet dabei einen hinteren (distalen) Teil und die Spritzenaufnahme 4 einen vorderen (proximalen) Teil der stiftartigen Spritzenvorrichtung 2. Die Spritzenaufnahme 4 ist durch einen mit dem Antriebsgehäuse 3 über ein Drehgelenk 7 verbundenen Bedienhebel 5, der an seinem von dem Antriebsgehäuse 3 wegweisenden Ende eine Kopfeinheit 8 aufweist, gebildet. Das Drehgelenk 7 ist dabei in einem hinteren Bereich des Antriebsgehäuses 3 angeordnet. Der Bedienhebel 5 ragt mit einem Betätigungsende 6 über das hintere Ende des Antriebsgehäuses 3 hinaus, so dass bei Drücken des Betätigungsendes 6 durch einen Bediener der Bedienhebel 5 von einer in Fig. 3 gezeigten geschlossenen Stellung in eine in Fig. 2 gezeigte offene bzw. obere Stellung gedreht wird.

In der in Fig. 2 gezeigten offenen Stellung kann eine Spritze 17 in die Spritzenaufnahme 4 eingebracht werden. Die Spritze 17 wird dabei auf ein Verbindungsstück 15, das an der in Richtung der Spritzenaufnahme 4 weisenden vorderen Seite des Antriebsgehäuses 3 angeordnet ist und etwas in das Innere der Spritze 17 bzw. den Spritzenkörper 18 eingreift, aufgesteckt. Auf den Verbindungsstutzen 15 ist ein Dichtring 16 aufgebracht durch den eine gas- oder luftdichte Verbindung zwischen dem Verbindungsstutzen 15 und der Spritze 17 bzw. dem Inneren des Spritzenkörpers 18 besteht.

Im geschlossenen Zustand der stiftartigen Spritzenvorrichtung 2, bei dem der Bedienhebel 5 in der in Fig. 3 gezeigten geschlossenen bzw. unteren Stellung ist, umgreift die Kopfeinheit 9 den in Richtung des Bedienhebels 5 weisenden oberen Bereich einer an dem vorderen (proximalen) Ende der Spritze 17 angeordneten Spritzenschulter 21, wobei der von der Kopfeinheit 9 gefasste Teil der Spritzenschulter 21 unmittelbar an der Kopfeinheit 9 anliegt. Die Spritze 17 wird dabei durch die Kopfeinheit 9 und das vordere Ende des Antriebsgehäuses 3, an dem der Spritzenflansch 20 anliegt, eingeklemmt. Ein Klemmen des Spritzenflansches 20, also eine Krafteinwirkung von dem vorderen Rand des Spritzenflansches 20 in Richtung des Antriebsgehäuses 3, erfolgt bei dem beschriebenen Ausführungsbeispiel nicht. Ein Brechen des Spritzenflansches 20 aufgrund von in diesem Bereich auftretenden Abweichungen, speziell eines leicht in Richtung des vorderen Endes der Spritze 17 geneigten Spritzenflansches 20, werden dadurch vermieden. Das Antriebsgehäuse 3 kann auch eine Flanschaufnahme aufweisen, die den Spritzenflansch 20 umfasst. Insbesondere kann das Antriebsgehäuse 3 auf der in Richtung der Spritzenaufnahme 4 weisenden Seite dabei ein nicht gezeigtes Dichtelement, insbesondere einen zweiten Dichtring, aufweisen, der an dem Spritzenflansch 20 anliegt und durch den eine gas- oder luftdichte Verbindung zwischen Spritzenflansch 20 und Antriebsgehäuse 3 gegeben ist. In dem in Fig. 3 gezeigten geschlossenen Zustand der stiftartigen Spritzenvorrichtung 2 ragt die Düse 22 der Spritze 17 dabei über das vordere Ende der Kopfeinheit 9 hinaus.

Bei einer vorzunehmenden Injektion, beispielsweise einer ophthalmologischen Injektion von Perfluorcarbonen ins Auge eines Patienten, ist die in die Spritzenaufnahme 4 eingebrachte Spritze 17 mit einer zu injizierenden Substanz gefüllt. Die zu injizierende Substanz ist dabei zwischen Düse 22 und Kolben 19 in dem Spritzenkörper 18 der Spritze 17 angeordnet. Bei einer vorzunehmenden Entnahme ist die in die Spritzenaufnahme 4 eingebrachte Spritze 17 hingegen nicht gefüllt. Der Kolben 19 ist dabei anfänglich bei oder nahe der Düse 22 angeordnet. Die stiftartige Spritzenvorrichtung 2, insbesondere die Spritzenaufnahme 4, ist entsprechend der Größe einer in die Spritzenaufnahme 4 einzubringenden Spritze 17 ausgestaltet. Beispielsweise kann die Spritzenaufnahme 4 zur Aufnahme von für die Injektion bzw. Entnahme bekannten Standardspritzen, insbesondere 10 ml-Spritzen, die z.B. bei der Injektion von Perfluorcarbonen zum Einsatz kommen, ausgestaltet sein.

Eine Injektion oder Entnahme erfolgt durch pneumatische Beaufschlagung des in dem Spritzenkörper 18 der Spritze 17 beweglich geführten Kolbens 19, insbesondere durch unmittelbares Beaufschlagen des Kolbens 19 mit Druck oder Unterdruck. Zur Beaufschlagung des Kolbens 19 mit Druck oder Unterdruck weist das Antriebsgehäuse 3 eine Druckversorgungseinheit und einen auf der Unterseite des Antriebsgehäuses 3 angeordneten Koppelanschluss 5 auf. Die Druckversorgungseinheit umfasst dabei einen mit dem Kolben 19 einer in die Spritzenaufnahme 4 eingebrachten Spritze 17 verbundenen Antriebskanal 10, der, wie in Fig. 5 gezeigt ist, durch den Verbindungsstutzen 15 und das Antriebsgehäuse 3 hindurch verläuft und mit dem Koppelanschluss 5 verbunden ist. Der Antriebskanal 10 kann dabei insbesondere durch ein oder mehrere in dem Antriebsgehäuse 3 verlaufende nicht gezeigte Schlauchelemente gebildet sein. Durch die externe Versorgungseinheit 24, die wie in Fig. 1 gezeigt ist, über den Schlauch 23 mit dem Koppelanschluss 5 verbunden ist, kann die Druckversorgungseinheit und damit der Kolben 19 mit Druck oder Unterdruck beaufschlagt werden. Die Betätigung der externen Versorgungseinheit 24 erfolgt über einen an dem vorderen Bereich auf dem Bedienhebel angeordneten, manuell durch einen Finger betätigbaren Taster 8, der über eine nicht gezeigte Betätigungsverbindung mit der Fördereinrichtung verbunden ist. Die Betätigung des Tasters 8 erfolgt dabei senkrecht zur Längsrichtung L der stiftartigen Spritzenvorrichtung 2.

Weiter umfasst die stiftartige Spritzenvorrichtung 2 einen Entlüftungskanal 12 mit einem ersten Ende, das mit dem Antriebskanal 10 verbunden ist, und einem zweiten Ende, das über eine Öffnung 13 mit der Umgebung verbunden ist. Die Öffnung 13 ist dabei, wie in Fig. 4 gezeigt ist, unmittelbar an dem Taster 8 genauer auf der dem Antriebsgehäuse 3 zugewandten Seite auf der Oberseite des Bedienhebels 5 angeordnet. Der Entlüftungskanal 12 verläuft, wie in Fig. 5 gezeigt ist, in dem Bedienhebel 5 von der Öffnung 13 bis an ein in dem hinteren Bereich des Bedienhebels 5 angeordnetes Verbindungsstück 14, von dem Verbindungsstück 14 in das Antriebsgehäuse 3 und von dort durch das Antriebsgehäuse 3 bis an den Antriebskanal 10. Der zwischen Verbindungsstück 14 und Antriebskanal 10 gebildete Abschnitt wird dabei bevorzugt durch ein nicht gezeigtes, mit dem Verbindungsstück 14 verbundenes Schlauchstück gebildet, das insbesondere ein Drehen des Bedienhebels 5 in die in Fig. 2 gezeigte geöffneten Stellung ermöglicht. Weiter kann die Verbindung zwischen dem Antriebskanal 10, insbesondere eines aus mehreren Schlauchstücken gebildeten Antriebskanals 10, und dem Entlüftungskanal 12 durch ein nicht gezeigtes, die einzelnen Schlauchstücke verbindendes T-Stück gebildet sein.

Die in Fig. 6 im Detail gezeigte externe Versorgungseinheit 24 umfasst ein quaderförmiges Gehäuse 25 mit einem in Fig. 1 gezeigten und in Fig. 6 weggelassenen Deckel 26 in dem eine elektrische Fördereinrichtung 27, vorliegend zwei Membranpumpen, zur Erzeugung von Druck oder Unterdruck, angeordnet ist. Weiter umfasst die externe Versorgungseinheit 24 eine an dem Gehäuse 25 angeordnete Stromversorgungseinheit 28, speziell einen Akkumulator. Die Stromversorgungseinheit 28 dient dabei zur Versorgung der elektrischen Fördereinrichtung 27 mit elektrischer Energie und kann über einen Ladeanschluss 29, beispielsweise einen USB-Anschluss, geladen werden. Weiter verfügt die externe Versorgungseinheit 24 über ein mit der elektrischen Fördereinrichtung 27 verbundenes Koppelstück 30 über das die externe Versorgungseinheit 24, wie in Fig. 1 gezeigt ist, über einen Schlauch 23 mit dem Koppelanschluss 11 der stiftartigen Spritzenvorrichtung 2 fluidisch verbindbar ist.

Fig. 7 zeigt das Spritzensystem aus Fig. 1, wobei die elektrische Fördereinrichtung 27 an einer skizzierter Armmanschette 32 befestigt ist. Lediglich der besseren Übersicht halber sind in Fig. 7 nicht alle Bezugszeichen des Spritzensystems 1 bzw. der stiftartigen Spritzenvorrichtung 2 und der externen Versorgungseinheit 24 eingezeichnet. Die Armmanschette 32 und die elektrische Fördereinrichtung 27 können bei einer Benutzung des Spritzensystems 1 beispielsweise von dem Kleidungsstück eines Bedieners, speziell den Ärmel eines OP-Kittels, verdeckt werden.

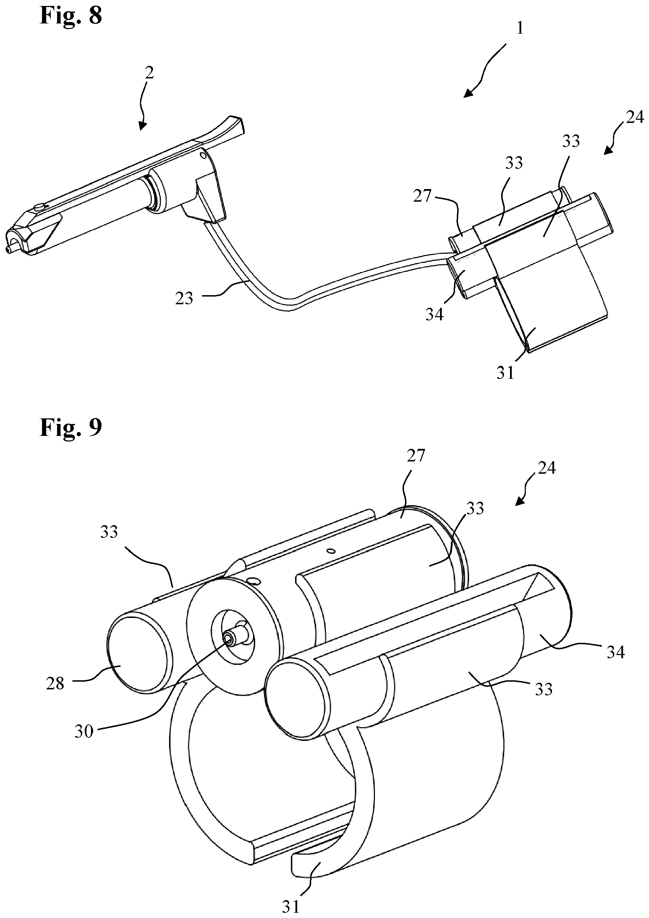

In Fig. 8 ist ein Spritzensystem 1 mit einer stiftartigen Spritzenvorrichtung 2 und einer externen Versorgungseinheit 24 gemäß einem zweiten Ausführungsbeispiel gezeigt. Das Spritzensystem 1 unterscheidet sich dabei von dem ersten Ausführungsbeispiel durch die Ausgestaltung der in Fig. 9 im Detail gezeigten externen Versorgungseinheit 24. Die stiftartige Spritzenvorrichtung 2 entspricht der stiftartigen Spritzenvorrichtung 2 des ersten Ausführungsbeispiels, wobei lediglich aus Übersichtsgründen in Fig. 8 nicht alle Bezugszeichen der stiftartigen Spritzenvorrichtung 2 eingezeichnet sind.

Die in Fig. 9 im Detail gezeigte externe Versorgungseinheit 24 des zweiten Ausführungsbeispiels ist als Armreif 31 ausgestaltet, der drei in Umfangsrichtung nebeneinander angeordnete Modulaufnahmen 33 aufweist. In der mittleren Modulaufnahme 33 ist dabei eine elektrische Fördereinrichtung 27, die auch in dieser Ausführungsform als eine Membranpumpe ausgestaltet ist, abnehmbar eingebracht. Die elektrische Fördereinrichtung 27 verfügt dabei ebenfalls über ein Koppelstück 30 über das die externe Versorgungseinheit 24, wie in Fig. 8 gezeigt ist, über einen Schlauch 23 mit dem Koppelanschluss 11 der stiftartigen Spritzenvorrichtung 2 verbindbar ist. In eine der beiden äußeren Modulaufnahmen 33 ist eine Stromversorgungseinheit 28, insbesondere ein Akkumulator, zur Versorgung der elektrischen Fördereinrichtung 27 mit elektrischer Energie angeordnet. Die Stromversorgungseinheit 28 kann über einen Ladeanschluss 29, beispielsweise einen USB-Anschluss, geladen werden. In die andere Modulaufnahme 33 ist ein Tuchspender 34 abnehmbar eingebracht. Von dem Tuchspender 34 kann dabei eine textile Abdeckung, insbesondere ein Tuch, zum Abdecken der externen Versorgungseinheit 24 bereitgestellt werden. Das Tuch kann dabei beispielsweise um den gesamten Armreif herum zurück zum Tuchspender 34 geführt und dort fixiert werden. Dadurch ist eine einfache und schnelle sterile Abdeckung der externen Versorgungseinheit 24 während des Einsatzes des Spritzensystems 1 möglich.

Die Bedienung und Funktion des Spritzensystems 1 wird im Folgenden anhand einer vorzunehmenden Injektion beschrieben. Dabei ist eine mit einer zu injizierenden Substanz gefüllte Spritze 17 in die Spritzenaufnahme 4 eingebracht und die stiftartige Spritzenvorrichtung 2 ist in der für den Betrieb vorgesehenen, in Fig. 3 gezeigten geschlossenen Stellung. Die stiftartige Spritzenvorrichtung 2 weist dabei in etwa die Größe einer menschlichen Hand auf und wird dabei wie ein Stift zwischen Daumen, Zeigefinger und Mittelfinger gehalten und geführt, wobei der Zeigefinger auf dem Bedienhebel 5 an- oder aufliegt. Die externe Versorgungseinheit 24 ist über die Armmanschette 32 oder den Armreif 31 an einem Arm des Bedieners angeordnet.

Bei der vorzunehmenden Injektion wird die elektrische Fördereinrichtung 27 der externen Versorgungseinheit 24 durch die Betätigung des Tasters 8 durch einen den Taster 8 betätigenden Finger des Bedieners, hier den Zeigefinger, aktiviert. Über den Schlauch 23 wird die Druckversorgungseinheit der stiftartigen Spritzenvorrichtung 2 mit Druck, hier mit Druckluft, beaufschlagt. Bei ordnungsgemäßer Handhabung der stiftartigen Spritzenvorrichtung 2 bzw. ordnungsgemäßer Betätigung des Tasters 8 wird die Öffnung 13 von dem den Taster 8 betätigenden Finger automatisch und zwangsweise verschlossen. Über den Antriebskanal 10 wird die als Antriebsmedium verwendete Druckluft in Richtung des Kolbens 19 der in die Spritzenaufnahme 4 eingebrachten Spritze 17 gefördert. Bei verschlossener Öffnung 13, die über den Entlüftungskanal 12 mit dem Antriebskanal 10 verbunden ist, erfolgt dabei eine Beaufschlagung des Kolbens 19 mit Druck. Der in dem Spritzenkörper 18 beweglich geführte Kolben 19 wird dadurch in Richtung der Düse 22 bewegt, wodurch die in der Spritze 17 enthaltene Substanz aus der Düse 22 bzw. einer daran angebrachten Kanüle ausgegeben wird. Bei nicht verschlossener Öffnung 13, beispielsweise bei nicht ordnungsgemäßer Betätigung des Tasters 8 oder bei Beenden der Betätigung des Tasters 8, steht der Antriebskanal 10 und somit der Kolben 19 mit der Umgebung in Verbindung. Eine (weitere) Beaufschlagung des Kolbens 19 mit Druck erfolgt aufgrund der durch die Öffnung 13 entweichenden Luft nicht. Bei einem Beenden der Betätigung des Tasters 8 ist dadurch ein unmittelbares Beenden der Injektion, insbesondere ohne Nachlaufeffekte wie ein Nachtropfen gewährleistet. Außerdem ist sichergestellt, dass bei einer nicht ordnungsgemäßen bzw. unkontrollierten Betätigung des Tasters 8 keine Injektion erfolgt.

Bei einer nicht näher beschriebenen, Entnahme einer Substanz, beispielsweise aus einem menschlichen Körper, wobei der Kolben 19 statt mit Druck mit Unterdruck beaufschlagt wird, gelten die obigen Ausführungen entsprechend.

Durch die Ausgestaltung des erfindungsgemäßen Spritzensystems 1 ist ein einfaches und sicheres Halten, Führen und Bedienen, der stiftartigen Spritzenvorrichtung 2, insbesondere unabhängig von der Größe der Hand eines Bedieners und somit eine sichere und gezielte Injektion oder Entnahme einer Substanz gegeben. Insbesondere ist die stiftartige Spritzenvorrichtung 2 aufgrund der Auslagerung der elektrischen Fördereinrichtung 27 und der Stromversorgungseinheit 28 sehr leicht und bietet somit einen hohen Komfort und eine angenehme, nicht ermüdende Benutzung der stiftartigen Spritzenvorrichtung 2. Weiter besteht die bei aus dem Stand der Technik bekannten Spritzensystemen vorliegende Gefahr einer möglichen Kontamination der zu injizierenden bzw. zu entnehmenden Substanz, die insbesondere durch eine zum Antrieb des Kolbens mit diesem verbundene Kolbenstange (geschmierte Mechanik) oder durch Umfüllen der zu injizierenden bzw. zu entnehmenden Substanz von einem externen Behälter in die Spritze bzw. umgekehrt gegeben ist, bei dem erfindungsgemäßen Spritzensystem 1 nicht. Die gezeigte stiftartige Spritzenvorrichtung 2 ermöglicht außerdem durch den drehbar an dem Antriebsgehäuse 3 angelenkten Bedienhebel 5 ein einfaches und schnelles Ein- und Ausbringen einer Spritze 17 in bzw. aus der Spritzenaufnahme 4. Insbesondere können bei der erfindungsgemäßen stiftartigen Spritzenvorrichtung 2 herkömmliche Standardspritzen (ohne Fingerflansch und Kolbenstange) verwendet werden. Die Spritzenaufnahme 4 sowie der Verbindungsstutzen 15 sind dabei entsprechend der zu verwendenden (Standard-) Spritzen 17 ausgestaltet.

Die Erfindung ist nicht auf die vorangehend beschriebenen Ausführungsbeispiele beschränkt. Beispielsweise ist auch eine Ausgestaltung möglich, bei der die elektrische Fördereinrichtung 27, speziell eine Membranpumpe, im Antriebsgehäuse 3 der Spritzenvorrichtung 2 angeordnet und Teil der Druckversorgungseinheit ist. Der Koppelanschluss 11 ist hierbei ein mit der elektrischen Fördereinrichtung 27 verbundener elektrischer Anschluss, der zur elektrischen Verbindung mit einer in der externen Versorgungseinheit 24 angeordneten Stromversorgungseinheit 28 dient. Anstelle eines Schlauchs 23 ist hierbei eine Kabelverbindung zur Stromversorgung eingesetzt.

Darüber hinaus sind auch andere Ausgestaltungen der externen Versorgungseinheit 24 und der stiftartigen Spritzenvorrichtung 2, beispielsweise eine Anordnung der Öffnung 13 auf der Oberseite des Tasters 8, möglich. Weiter ist es auch möglich, dass die Spritzenaufnahme 4 temperierbar, insbesondere beheizbar, ausgestaltet ist, wofür eine Temperiereinheit in der Spritzenvorrichtung 2 angeordnet und über die Stromversorgungseinheit 28 oder eine weitere externe Stromversorgungseinheit, insbesondere eine weitere an der externen Versorgungseinheit 24 angeordnete externe Stromversorgungseinheit, mit elektrischer Energie versorgt wird.

Weiter kann die Spritzenaufnahme 4 auch einen Splitterschutz, also einen Schutz vor Splittern bei einer auftretenden Beschädigung der Spritze 17, aufweisen. Beispielsweise kann der Splitterschutz durch ein an dem Bedienhebel angeordnetes Plexiglaselement, welches die Spritze 17 abdeckt oder in welches die Spritze 17 eingebracht wird, gebildet sein. Alternativ oder zusätzlich kann die Spritze 17 ein umlaufendes Etikett, speziell ein den Spritzenkörper 18 umlaufendes Etikett aufweisen, das ein Splittern der Spritze 17 oder zumindest ein Ablösen von Splittern verhindert.

### Bezugszeichen

- 1: Spritzensystem
- 2: Stiftartige Spritzenvorrichtung
- 3: Antriebsgehäuse
- 4: Spritzenaufnahme
- 5: Bedienhebel
- 6: Betätigungsende
- 7: Drehgelenk
- 8: Taster
- 9: Kopfeinheit
- 10: Antriebskanal
- 11: Koppelanschluss
- 12: Entlüftungskanal
- 13: Öffnung
- 14: Verbindungsstück
- 15: Verbindungsstutzen
- 16: Dichtring
- 17: Spritze
- 18: Spritzenkörper
- 19: Kolben
- 20: Spritzenflansch
- 21: Spritzenschulter
- 22: Düse
- 23: Schlauch
- 24: Externe Versorgungseinheit
- 25: Gehäuse
- 26: Deckel
- 27: Elektrische Fördereinrichtung
- 28: Stromversorgungseinheit
- 29: Ladeanschluss
- 30: Koppelstück
- 31: Armreif
- 32: Armmanschette
- 33: Modulaufnahme
- 34: Tuchspender
- L: Längsrichtung

## Patentansprüche

1. Stiftartige Spritzenvorrichtung (2) für medizinische und insbesondere für ophthalmologische Zwecke, insbesondere zum Einbringen von Fluiden in das menschliche oder tierische Auge, umfassend ein Antriebsgehäuse (3), eine an das Antriebsgehäuse (3) anschließende Spritzenaufnahme (4) zur Aufnahme einer Spritze (17), welche einen Spritzenkörper (18) und einen in dem Spritzenkörper (18) beweglich geführten Kolben (19) aufweist, und einen manuell betätigbaren Taster (8) zur Aktivierung einer elektrischen Fördereinrichtung, wobei das Antriebsgehäuse (3) eine Druckversorgungseinheit zur pneumatischen Beaufschlagung des Kolbens (19) einer in die Spritzenaufnahme (4) eingebrachten Spritze (17) mit Druck oder Unterdruck und einen Koppelanschluss (11) zur pneumatischen und/oder elektrischen Verbindung der Druckversorgungseinheit mit einer externen Versorgungseinheit (24) aufweist, welche entfernt vom Antriebsgehäuse (3) angeordnet ist, wobei die stiftartige Spritzenvorrichtung (2) selbst keine integrierte Stromversorgungseinheit (28), insbesondere keine Batterie oder keinen Akkumulator, und/oder keine elektrische Fördereinrichtung (27) aufweist, und die Druckversorgungseinheit einen Antriebskanal (10), der mit dem Kolben (19) einer in die Spritzenaufnahme (4) eingebrachten Spritze (17) verbindbar ist, umfasst und die stiftartige Spritzenvorrichtung (2) einen Entlüftungskanal (12) mit einem ersten Ende, das mit dem Antriebskanal (10) verbunden ist, und einem zweiten Ende, das über eine Öffnung (13) mit der Umgebung verbunden ist, aufweist, wobei die Öffnung (13) bei Betätigung des Tasters (8) von einem den Taster (8) betätigenden Finger verschließbar ist.

2. Stiftartige Spritzenvorrichtung (2) nach Anspruch 1, wobei die Öffnung (13) direkt an oder unmittelbar neben dem Taster (8) angeordnet ist und dadurch unmittelbar durch einen den Taster (8) betätigenden Finger, insbesondere durch den Finger selbst, verschließbar ist.

3. Stiftartige Spritzenvorrichtung (2) nach einem der voranstehenden Ansprüche, wobei die externe Versorgungseinheit (24) eine elektrische Fördereinrichtung (27) zur Versorgung der Druckversorgungseinheit mit Druck oder Unterdruck aufweist, wobei insbesondere durch Betätigen des Tasters (8) eine Aktivierung der elektrischen Fördereinrichtung (27) erfolgt.

4. Stiftartige Spritzenvorrichtung (2) nach Anspruch 3, wobei die elektrische Fördereinrichtung (27) eine Membranpumpe, insbesondere eine über den Taster (8) betätigbare Membranpumpe, umfasst.

5. Stiftartige Spritzenvorrichtung (2) nach Anspruch 1 oder 2, wobei die Druckversorgungseinheit eine mit dem Antriebskanal (10) verbundene elektrische Fördereinrichtung (27), insbesondere eine Membranpumpe, umfasst, wobei durch Betätigen des Tasters (8) eine Aktivierung der elektrischen Fördereinrichtung (27) und ein Beaufschlagen des Antriebskanals (10) mit Druck oder Unterdruck erfolgt.

6. Stiftartige Spritzenvorrichtung (2) nach einem der voranstehenden Ansprüche, wobei der Taster (8) nahe dem vorderen bzw. dem von dem Antriebsgehäuse (10) wegweisenden Ende der stiftartigen Spritzenvorrichtung (2) angeordnet ist.

7. Stiftartige Spritzenvorrichtung (2) nach einem der voranstehenden Ansprüche, wobei die Spritzenaufnahme (4) einen schwenkbar mit dem Antriebsgehäuse (3) verbundenen Bedienhebel (5) umfasst, der bevorzugt über ein an einem von der Spritzenaufnahme (4) wegweisenden Ende des Antriebsgehäuses (3) angeordneten Drehgelenk (7) mit dem Antriebsgehäuse (3) verbunden ist, wobei insbesondere der Entlüftungskanal (12) zumindest teilweise in dem Bedienhebel (5) verläuft und/oder der Taster (8) und die Öffnung (13) an bzw. auf dem Bedienhebel (5) angeordnet sind.

8. Stiftartige Spritzenvorrichtung (2) nach einem der voranstehenden Ansprüche, wobei die Spritzenaufnahme (4) eine Kopfeinheit (9), insbesondere eine an einem Bedienhebel (5) angeordnete Kopfeinheit (9), umfasst, die eine Spritzenschulter (21) einer in die Spritzenaufnahme (4) eingebrachten Spritze (17) zumindest teilweise fasst, wobei zumindest ein Teil der Spritzenschulter (21) im Betriebszustand der stiftartigen Spritzenvorrichtung (2) unmittelbar an der Kopfeinheit (9) anliegt, wobei insbesondere eine in die Spritzenaufnahme (4) eingebrachte Spritze (17) im Betriebszustand der stiftartigen Spritzenvorrichtung (2) durch die Kopfeinheit (9) und die in Richtung der Spritzenaufnahme (4) weisende Seite des Antriebsgehäuses (10) eingeklemmt und fixiert ist, wobei insbesondere kein Klemmen eines Spritzenflansches (21) der Spritze (17) erfolgt.

9. Stiftartige Spritzenvorrichtung (2) nach einem der voranstehenden Ansprüche, wobei die stiftartige Spritzenvorrichtung (2) eine Temperiereinheit zur Temperierung einer in einer eingebrachten Spritze (17) enthaltenen Substanz aufweist, wobei die Temperiereinheit bevorzugt in der Spritzenaufnahme (4) angeordnet ist.

10. Stiftartige Spritzenvorrichtung (2) nach einem der voranstehenden Ansprüche, wobei in die Spritzenaufnahme (4) eine Spritze (17) eingebracht wird, wobei die Spritze (17) einen Spritzenkörper (18) und einen in dem Spritzenkörper (18) beweglich geführten Kolben (19) aufweist.

11. Spritzensystem (1) umfassend eine stiftartige Spritzenvorrichtung (2) nach einem der voranstehenden Ansprüche sowie eine entfernt von der stiftartigen Spritzenvorrichtung (2) angeordnete externe Versorgungseinheit (24), die eine Stromversorgungseinheit (28), insbesondere eine Batterie oder einen Akkumulator, und/oder eine elektrische Fördereinrichtung (27), insbesondere eine Membranpumpe, umfasst.

12. Spritzensystem (1) nach Anspruch 11, wobei die externe Versorgungseinheit (24) eine elektrische Fördereinrichtung (27) umfasst, die über eine Betätigungsverbindung mit dem Taster (8) der stiftartigen Spritzenvorrichtung (2) verbunden ist.

13. Spritzensystem (1) nach Anspruch 11 bis 12 wobei die externe Versorgungseinheit (24) eine Befestigungseinrichtung, insbesondere eine Armmanschette (32) oder einen Armreif (31) umfasst, mit der die externe Versorgungseinheit (24) an einem Arm eines Bedieners befestigbar ist und/oder dass die externe Versorgungseinheit (24) einen Tuchspender (34), insbesondere einen Tuchspender (34) mit einer bevorzugt textilen Abdeckung zur Abdeckung der externen Versorgungseinheit (24), umfasst.

14. Spritzensystem (1) nach einem der Ansprüche 11 bis 13, wobei die externe Versorgungseinheit (24) eine Steuereinheit zur Steuerung der externen Versorgungseinheit (24), insbesondere zur Steuerung einer elektrischen Fördereinrichtung (27) der externen Versorgungseinheit (24), aufweist und/oder dass die externe Versorgungseinheit (24) eine Anzeigeeinheit zur Anzeige von Parametern des Spritzensystems (1), insbesondere des Zustands der Stromversorgungseinheit (28) und/oder der Betriebsparameter der elektrischen Fördereinrichtung (27) und/oder des auf den Kolben (19) einer in die Spritzenaufnahme (4) eingebrachten Spritze (17) wirkenden Drucks oder Unterdrucks, aufweist.

## Claims

1. Pen-like syringe device (2) for medical and in particular for ophthalmological purposes, in particular for introducing fluids into the human or animal eye, comprising a drive housing (3), a syringe receptacle (4) adjoining the drive housing (3) for receiving a syringe (17), which has a syringe body (18) and a plunger (19) movably guided in the syringe body (18), and a manually operable push button (8) for activating an electrical delivery unit, wherein the drive housing (3) has a pressure supply unit for pneumatically applying pressure or negative pressure to the plunger (19) of a syringe (17) inserted into the syringe receptacle (4) and a coupling connection (11) for pneumatically and/or electrically connecting the pressure supply unit to an external supply unit (24) which is arranged away from the drive housing (3), the pen-like syringe device (2) itself having no integrated power supply unit (28), in particular no battery or no accumulator, and/or no electrical delivery unit (27), and the pressure supply unit comprising a drive channel (10) which can be connected to the plunger (19) of a syringe (17) which is inserted into the syringe receptacle (4) and the pen-like syringe device (2) comprises a vent channel (12) having a first end connected to the drive channel (10) and a second end connected to the environment via an opening (13), the opening (13) being closable by a finger actuating the push button (8) upon actuation of the push button (8).

2. Pen-like syringe device (2) according to claim 1, wherein the opening (13) is arranged directly on or directly next to the push button (8) and can thereby be closed directly by a finger actuating the push button (8), in particular by this finger itself.

3. Pen-like syringe device (2) according to one of the preceding claims, wherein the external supply unit (24) comprises an electrical delivery unit (27) for supplying the pressure supply unit with pressure or negative pressure, wherein in particular activation of the electrical delivery unit (27) is effected by actuating the push button (8).

4. Pen-like syringe device (2) according claim 3, wherein the electrical delivery unit (27) comprises a diaphragm pump, in particular a diaphragm pump operable via the push button (8).

5. Pen-like syringe device (2) according to claim 1 or 2, wherein the pressure supply unit comprises an electrical delivery unit (27), in particular a diaphragm pump, connected to the drive channel (10), the electrical delivery unit (27) being activated and the drive channel (10) being applied with pressure or negative pressure by actuating the push button (8).

6. Pen-like syringe device (2) according to one of the preceding claims, wherein the push button (8) is arranged near the front end or the end facing away from the drive housing (10) of the pen-like syringe device (2).

7. Pen-like syringe device (2) according to one of the preceding claims, wherein the syringe receptacle (4) comprises an operating lever (5) which is pivotably connected to the drive housing (3) and is preferably connected to the drive housing (3) via a swivel joint (7) arranged at an end of the drive housing (3) facing away from the syringe receptacle (4), in particular wherein the vent channel (12) runs at least partially in the operating lever (5) and/or the push button (8) and the opening (13) are arranged on or on top of the operating lever (5).

8. Pen-like syringe device (2) according to one of the preceding claims, wherein the syringe receptacle (4) comprises a head unit (9), in particular a head unit (9) arranged on an operating lever (5), which at least partially grips a syringe shoulder (21) of a syringe (17) inserted into the syringe receptacle (4), at least part of the syringe shoulder (21) bearing directly against the head unit (9) in the operating state of the pen-like syringe device (2), wherein in particular a syringe (17) inserted into the syringe receptacle (4) is clamped and fixed in the operating state of the pen-like syringe device (2) by the head unit (9) and the side of the drive housing (10) facing in the direction of the syringe receptacle (4), in particular that no clamping of a syringe flange (21) of the syringe (17) takes place.

9. Pen-like syringe device (2) according to one of the preceding claims, wherein the pen-like syringe device (2) has a temperature control unit for controlling the temperature of a substance contained in an inserted syringe (17), the temperature control unit preferably being arranged in the syringe receptacle (4).

10. Pen-like syringe device (2) according to one of the preceding claims, wherein a syringe (17) is inserted into the syringe receptacle (4), the syringe (17) comprising a syringe body (18) and a plunger (19) movably guided in the syringe body (18).

11. Syringe system (1) comprising a pen-like syringe device (2) according to one of the preceding claims and an external supply unit (24) arranged away from the pen-like syringe device (2), which comprises a power supply unit (28), in particular a battery or an accumulator, and/or an electrical delivery unit (27), in particular a diaphragm pump

12. Syringe system (1) according to claim 11, wherein the external supply unit (24) comprises an electrical delivery unit (27) which is connected to the push button (8) of the pen-like syringe device (2) via an actuating connection.

13. Syringe system (1) according to claim 11 to 12, wherein the external supply unit (24) comprises a fastening device, in particular an wrist band (32) or an arm cuff (31), with which the external supply unit (24) can be fastened to an arm of an operator and/or wherein the external supply unit (24) comprises a towel dispenser (34), in particular a towel dispenser (34) with a preferably textile cover for covering the external supply unit (24).

14. Syringe system (1) according to any one of claims 11 to 13, wherein the external supply unit (24) has a control unit for controlling the external supply unit (24), in particular for controlling an electrical delivery unit (27) of the external supply unit (24), and/or that the external supply unit (24) has a display unit for displaying parameters of the syringe system (1), in particular the state of the power supply unit (28) and/or the operating parameters of the electrical delivery unit (27) and/or the pressure or negative pressure acting on the plunger (19) of a syringe (17) inserted into the syringe receptacle (4).

## Revendications

1. Dispositif à seringue de type stylo (2) pour des applications médicales et en particulier pour des applications en ophtalmologie, en particulier pour introduire des fluides dans l'œil chez l'homme ou chez l'animal, comprenant un boîtier d'entraînement (3), un logement de seringue (4) se raccordant au boîtier d'entraînement (3), destiné à loger une seringue (17), laquelle présente un corps de seringue (18) et un piston (19) guidé de manière mobile dans le corps de seringue (18), et un bouton (8) pouvant être actionné manuellement destiné à activer un équipement de refoulement électrique, dans lequel le boîtier d'entraînement (3) présente une unité d'alimentation en pression destinée à soumettre de manière pneumatique le piston (19) d'une seringue (17) introduite dans le logement de seringue (4) à l'action d'une pression ou d'une dépression et un raccord d'accouplement (11) destiné à relier de manière pneumatique et/ou électrique l'unité d'alimentation en pression à une unité d'alimentation externe (24), laquelle est disposée de manière éloignée du boîtier d'entraînement (3), dans lequel le dispositif à seringue de type stylo (2) ne présente lui-même aucune unité d'alimentation en courant intégrée (28), en particulier aucune batterie ni aucun accumulateur, et/ou aucun équipement de refoulement électrique (27), et l'unité d'alimentation en pression comprend un canal d'entraînement (10), qui peut être relié au piston (19) d'une seringue (17) introduite dans le logement de seringue (4) et le dispositif à seringue de type stylo (2) présente un canal d'évacuation d'air (12) avec une première extrémité, qui est reliée au canal d'entraînement (10), et une deuxième extrémité, qui est reliée à l'environnement par l'intermédiaire d'un orifice (13), dans lequel l'orifice (13) peut être fermé lors de l'actionnement du bouton (8) par un doigt actionnant le bouton (8).

2. Dispositif à seringue de type stylo (2) selon la revendication 1, dans lequel l'orifice (13) est disposé directement sur ou immédiatement à côté du bouton (8) et peut ainsi être fermé immédiatement par un doigt actionnant le bouton (8), en particulier par le doigt lui-même.

3. Dispositif à seringue de type stylo (2) selon l'une quelconque des revendications précédentes, dans lequel l'unité d'alimentation externe (24) présente un équipement de refoulement électrique (27) destiné à alimenter l'unité d'alimentation en pression en pression ou en dépression, dans lequel en particulier une activation de l'équipement de refoulement électrique (27) a lieu en particulier en actionnant le bouton (8).

4. Dispositif à seringue de type stylo (2) selon la revendication 3, dans lequel
l'équipement de refoulement électrique (27) comprend une pompe à membrane, en particulier une pompe à membrane pouvant être actionnée par l'intermédiaire du bouton (8).

5. Dispositif à seringue de type stylo (2) selon la revendication 1 ou 2, dans lequel
l'unité d'alimentation en pression comprend un équipement de refoulement électrique (27) relié au canal d'entraînement (10), en particulier une pompe à membrane, dans lequel une activation de l'équipement de refoulement électrique (27) et une action exercée par une pression ou dépression sur le canal d'entraînement (10) ont lieu en actionnant le bouton (8).

6. Dispositif à seringue de type stylo (2) selon l'une quelconque des revendications précédentes, dans lequel
le bouton (8) est disposé à proximité de l'extrémité, avant ou pointant de manière à s'éloigner du boîtier d'entraînement (10), du dispositif à seringue de type stylo (2).

7. Dispositif à seringue de type stylo (2) selon l'une quelconque des revendications précédentes, dans lequel
le logement de seringue (4) comprend un levier d'utilisation (5) relié de manière pivotante au boîtier d'entraînement (3), qui est relié au boîtier d'entraînement (3) de manière préférée par l'intermédiaire d'une articulation rotative (7) disposée sur une extrémité, pointant de manière à s'éloigner du logement de seringue (4), du boîtier d'entraînement (3), dans lequel en particulier le canal d'évacuation de l'air (12) s'étend au moins en partie dans le levier d'utilisation (5) et/ou le bouton (8) et l'orifice (13) sont disposés sur le ou au-dessus du levier d'utilisation (5) .

8. Dispositif à seringue de type stylo (2) selon l'une quelconque des revendications précédentes, dans lequel le logement de seringue (4) comprend une unité de tête (9), en particulier une unité de tête (9) disposée sur un levier d'utilisation (5), qui saisit au moins en partie un épaulement de seringue (21) d'une seringue (17) introduite dans le logement de seringue (4), dans lequel au moins une partie de l'épaulement de seringue (21) repose immédiatement sur l'unité de tête (9) dans l'état de fonctionnement du dispositif à seringue de type stylo (2), dans lequel en particulier une seringue (17) introduite dans le logement de seringue (4) est coincée et bloquée dans l'état de fonctionnement du dispositif à seringue de type stylo (2) par l'unité de tête (9) et le côté, pointant en direction du logement de seringue (4), du boîtier d'entraînement (10), dans lequel en particulier aucun serrage d'une bride de seringue (21) de la seringue (17) n'a lieu.

9. Dispositif à seringue de type stylo (2) selon l'une quelconque des revendications précédentes, dans lequel le dispositif à seringue de type stylo (2) présente une unité de thermorégulation pour thermoréguler une substance contenue dans une seringue (17) introduite, dans lequel l'unité de thermorégulation est disposée de manière préférée dans le logement de seringue (4).

10. Dispositif à seringue de type stylo (2) selon l'une quelconque des revendications précédentes, dans lequel une seringue (17) est introduite dans le logement de seringue (4), dans lequel la seringue (17) présente un corps de seringue (18) et un piston (19) guidé de manière mobile dans le corps de seringue (18).

11. Système à seringue (1) comprenant un dispositif à seringue de type stylo (2) selon l'une quelconque des revendications précédentes ainsi qu'une unité d'alimentation externe (24) disposée de manière éloignée du dispositif à seringue de type stylo (2), qui comprend une unité d'alimentation en courant (28), en particulier une batterie ou un accumulateur, et/ou un équipement de refoulement électrique (27), en particulier une pompe à membrane.

12. Système à seringue (1) selon la revendication 11, dans lequel l'unité d'alimentation externe (24) comprend un équipement de refoulement électrique (27), qui est relié au bouton (8) du dispositif à seringue de type stylo (2) par l'intermédiaire d'une liaison d'actionnement.

13. Système à seringue (1) selon la revendication 11 à 12, dans lequel
l'unité d'alimentation externe (24) comprend un équipement de fixation, en particulier un brassard (32) ou un bracelet (31), par lequel l'unité d'alimentation externe (24) peut être fixée sur un bras d'un utilisateur, et/ou que l'unité d'alimentation externe (24) comprend un distributeur d'essuie-mains (34), en particulier un distributeur d'essuie-mains (34) avec un recouvrement de manière préférée textile pour recouvrir l'unité d'alimentation externe (24).

14. Système à seringue (1) selon l'une quelconque des revendications 11 à 13, dans lequel
l'unité d'alimentation externe (24) présente une unité de commande destinée à commander l'unité d'alimentation externe (24), en particulier destinée à commander un équipement de refoulement électrique (27) de l'unité d'alimentation externe (24), et/ou que l'unité d'alimentation externe (24) présente une unité d'affichage destinée à afficher des paramètres du système à seringue (1), en particulier de l'état de l'unité d'alimentation en courant (28) et/ou des paramètres de fonctionnement de l'équipement de refoulement électrique (27) et/ou de la pression ou dépression agissant sur le piston (19) d'une seringue (17) introduite dans le logement de seringue (4).
